# EUROPEAN PATENT APPLICATION

(11) **EP 0 586 011 A2**
(43) Date of publication of application: **09.03.1994**
(21) Application number: 93202496.1
(22) Date of filing: 25.08.1993
(51) Int. Cl.: C12Q 1/68, C12Q 1/70

(54) **Methods for production and amplification of nucleic acids**

(30) Priority: 28.08.1992 US 937133
(71) Applicant: EASTMAN KODAK COMPANY, Rochester, New York 14650-2201 (US)
(72) Inventor: Bergmeyer, Lynn, c/oEASTMAN KODAK COMPANY, Rochester, New York 14650-2201 (US); Oakes, Fred Terry, c/oEASTMAN KODAK COMPANY, Rochester, New York 14650-2201 (US)
(74) Representative: Aufflick, James Neil

(57) **Abstract**

A target nucleic acid can be amplified by PCR in a sensitive fashion using two primers for the nucleic acid, one or more deoxyribonucleotide-5'-triphosphates, a DNA polymerase and a DNA polymerase cofactor. Both of the primers are labeled with the same detection moiety, such as a radioisotope, enzyme, avidin or biotin. The labeled primers can be supplied as part of a test kit.

SEQUENCE LISTING
INFORMATION FOR SEQ ID NO:1:
(i) SEQUENCE CHARACTERISTICS:
(A) LENGTH: 25 nucleotides
(B) TYPE: Nucleic acid
(C) STRANDEDNESS: Single
(D) TOPOLOGY: Linear
(ii) MOLECULE TYPE: Primer
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:1:
CAGCACCATC CTCCTCTTCC TCTGG 25

INFORMATION FOR SEQ ID NO:2:
(i) SEQUENCE CHARACTERISTICS:
(A) LENGTH: 25 nucleotides
(B) TYPE: Nucleic acid
(C) STRANDEDNESS: Single
(D) TOPOLOGY: Linear
(ii) MOLECULE TYPE: Primer
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:2:
GAGGCTATTG TAGCCTACAC TTTGG 25

INFORMATION FOR SEQ ID NO:3:
(i) SEQUENCE CHARACTERISTICS:
(A) LENGTH: 25 nucleotides
(B) TYPE: Nucleic acid
(C) STRANDEDNESS: Single
(D) TOPOLOGY: Linear
(ii) MOLECULE TYPE: Primer
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:3:
GTCGCCTGCA CTGCCAGGTG CTTCG 25

INFORMATION FOR SEQ ID NO:4:
(i) SEQUENCE CHARACTERISTICS:
(A) LENGTH: 25 nucleotides
(B) TYPE: Nucleic acid
(C) STRANDEDNESS: Single
(D) TOPOLOGY: Linear
(ii) MOLECULE TYPE: Primer
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:4:
CACCACGCAG CGGCCCTTGA TGTTT 25

INFORMATION FOR SEQ ID NO:5:
(i) SEQUENCE CHARACTERISTICS:
(A) LENGTH: 30 nucleotides
(B) TYPE: Nucleic acid
(C) STRANDEDNESS: Single
(D) TOPOLOGY: Linear
(ii) MOLECULE TYPE: Probe oligonucleotide
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:5:
GGTGTCACCC CCAGAGTCCC CTGTACCCGC 30

INFORMATION FOR SEQ ID NO:6:
(i) SEQUENCE CHARACTERISTICS:
(A) LENGTH: 30 nucleotides
(B) TYPE: Nucleic acid
(C) STRANDEDNESS: Single
(D) TOPOLOGY: Linear
(ii) MOLECULE TYPE: Probe oligonucleotide
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:6:
GTGGAAGGCG GCTCGCTGGA AGCCGGTCGT 30

INFORMATION FOR SEQ ID NO:7:
(i) SEQUENCE CHARACTERISTICS:
(A) LENGTH: 30 nucleotides
(B) TYPE: Nucleic acid
(C) STRANDEDNESS: Single
(D) TOPOLOGY: Linear
(ii) MOLECULE TYPE: Probe oligonucleotide
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:7:
GACACAGTGT CCTCCCGCTC CTCCTGAGCA 30

INFORMATION FOR SEQ ID NO:8:
(i) SEQUENCE CHARACTERISTICS:
(A) LENGTH: 30 nucleotides
(B) TYPE: Nucleic acid
(C) STRANDEDNESS: Single
(D) TOPOLOGY: Linear
(ii) MOLECULE TYPE: Probe oligonucleotide
(iii) HYPOTHETICAL: No
(iv) ANTI-SENSE: No
(vi) ORIGINAL SOURCE: Synthetically prepared
(vii) IMMEDIATE SOURCE: Synthetically
prepared
(x) PUBLICATION INFORMATION: None
(xi) SEQUENCE DESCRIPTION: SEQ ID NO:8:
GAACCGAGGG CCGGCTCACC TCTATGTTGG 30

## Description

This invention relates to methods for production, amplification and detection of a target nucleic acid using polymerase chain reaction (PCR) and two labeled primers. It also relates to a test kit useful in such methods, the test kit containing the labeled primers and other reagents needed for PCR.

Technology to detect minute quantities of nucleic acids has advanced rapidly over the last two decades including the development of highly sophisticated hybridization assays using probes in amplification techniques such as PCR. Researchers have readily recognized the value of such technology to detect diseases and genetic features in human or animal test specimens. The use of probes and primers in such technology is based upon the concept of complementarity, that is the bonding of two strands of a nucleic acid by hydrogen bonds between complementary nucleotides (also known as nucleotide pairs).

PCR is a significant advance in the art to allow detection of very small concentrations of a targeted nucleic acid. The details of PCR are described, for example, in US-A-4,683,195, US-A-4,683,202 and US-A-4,965,188, although there is a rapidly expanding volume of literature in this field. Without going into extensive detail, PCR involves hybridizing primers to the strands of a targeted nucleic acid (considered "templates") in the presence of a polymerization agent (such as a DNA polymerase) and deoxyribonucleoside triphosphates under the appropriate conditions. The result is the formation of primer extension products along the templates, the products having added thereto nucleotides which are complementary to the templates.

Once the primer extension products are denatured, and one copy of the templates has been prepared, the cycle of priming, extending and denaturation can be carried out as many times as desired to provide an exponential increase in the amount of nucleic acid which has the same sequence as the target nucleic acid. In effect, the target nucleic acid is duplicated (or "amplified") many times so that it is more easily detected.

It has become conventional to label one of the primers used in PCR with a suitable detectable moiety which can then be used to detect the amplified product. Useful labels include radioisotopes, fluorescent moieties, enzymes or specific binding moieties such as avidin or biotin, as noted for example in US-A-4,879,214.

EP-A-0 443 748 mentions the possible use of the same or different labels on the primers in the amplification of genomic DNA, but fails to demonstrate how this would be done or what results it might provide.

There is a continued need to improve the sensitivity of PCR in order to detect the smallest concentrations of target nucleic acids in a specimen.

According to the present invention the sensitivity of PCR has been improved with a method for the production of a target nucleic acid comprising:
amplifying the denatured strands of a target nucleic acid in the presence of two primers which are complementary to the complementary strands of the target nucleic acid, one or more deoxyribonucleotide-5'-triphosphates, a DNA polymerase and a DNA polymerase cofactor,
both of the primers being labeled with the same detection moiety.

This invention also provides a method for the amplification of a target nucleic acid comprising:
A) contacting the denatured strands of a target nucleic acid in the presence of polymerase chain reaction reagents comprising: two primers which are complementary to the complementary strands of the target nucleic acid, one or more deoxyribonucleotide-5'-triphosphates, a DNA polymerase and a DNA polymerase cofactor,
   both of the primers being labeled with the same detection moiety, and
B) forming primer extension products of the target nucleic acid strands with the labeled primers.

Moreover, this invention also provides a method for the detection of a target nucleic acid comprises:
A) contacting the denatured strands of a target nucleic acid with polymerase chain reaction reagents comprising: two primers which are complementary to the complementary strands of the target nucleic acid, one or more deoxyribonucleotide-5'-triphosphates, a DNA polymerase and a DNA polymerase cofactor,
   both of the primers being labeled with the same detection moiety,
B) forming primer extension products of the target nucleic acid strands with the labeled primers,
C) denaturing the strands of the resulting primer extension products,
D) repeating steps B) and C) as a PCR cycle at least one more time, and
E) detecting at least one of the denatured strands after the last PCR cycle.

This invention also provides a kit for polymerase chain reaction comprises, in separate packaging:
a) a set of two primers complementary to the complementary strands of a target nucleic acid, and
b) at least one additional PCR reagent, the kit characterized wherein both of the primers are labeled with the same detection moiety.

The present invention provides improved sensitivity in PCR through the use of two labeled primers for each nucleic acid sequence of a target nucleic acid being amplified or detected. The labels on both primers are used for detection of the amplified target nucleic acid.

The general principles and conditions for amplification and detection of nucleic acids using polymerase chain reaction are quite well known, the details of which are provided in numerous references including US-A-4,683,195, US-A-4,683,202, US-A-4,965,188 and WO-A-91/12342 and by Guatelli et al, Clin.Microbiol.Rev., 2(2), pp. 217-226 (1989). In view of the teaching in the art and the specific teaching provided herein, a worker skilled in the art should have no difficulty in practicing the present invention by making the adjustments taught herein to accomplish amplification of a target nucleic acid using two labeled primers.

The present invention is directed to the amplification or detection of one or more specific nucleic acid sequences present in one or more target nucleic acids in a test specimen. Such specimens can include cellular or viral material, hair, body fluids or other materials containing genetic DNA or RNA which can be detected. While the primary purpose of detection is diagnostic in nature, the invention can also be used to improve the efficiency of cloning DNA or messenger RNA, or for obtaining large amounts of the desired sequence from a mixture of nucleic acids resulting from chemical synthesis.

The present invention is especially useful for producing, in exponential quantities relative to the number of reaction steps involved, at least one specific nucleic acid sequence associated with an infectious agent. The product will be a discrete nucleic acid duplex with termini corresponding to the ends of the specific primers employed. Any source of nucleic acid, purified or not, can be utilized as the starting material if it is known to or suspected of containing the specific nucleic acid sequence targeted for detection. Moreover, a plurality of target nucleic acids can be amplified and detected simultaneously by using a corresponding set of labeled primers and detection means for each specific nucleic acid. Multiple sequences in the same nucleic acid can also be amplified and detected.

Nucleic acids to be detected can be obtained from various sources including plasmids, naturally occurring DNA or RNA from any source (such as bacteria, yeast, viruses, plants and higher animals, humans). It may be extracted from various tissues including blood, peripheral blood mononuclear cells (PBMC), tissue material or other sources known in the art using known procedures. The present invention is particularly useful for the amplification and detection of target nucleic acids found in bacterial DNA, fungal DNA, viral RNA, or DNA found in bacterial or virus-infected cells.

The method described herein can be used to provide the detection of specific nucleic acid sequences associated with infectious diseases, genetic disorders or cellular disorders such as cancers or any other disease state not specifically included in these categories. It may also be used in forensic investigations and DNA typing. For purposes of this invention, genetic diseases include specific deletions or mutations in genomic DNA from any organism, such as sickle cell anemia, cystic fibrosis, α-thalassemia, β-thalessemia and others readily apparent to one skilled in the art. Human Leukocyte Antigen (HLA) can be categorized with the present invention. Bacteria which can be detected include, but are not limited to, bacteria which may be found in the blood, Salmonella, Streptococcus species, Chlamydia species, Gonococcus species, Mycobacterium species (such as *Mycobacterium tuberculosis* and *Mycobacterium avium* complex), Mycoplasma species (such as *Mycoplasma Haemophilus influenzae* and *Mycoplasma pneumoniae*), *Legionella pneumophila, Borrelia burgdorferei, Pneumocystis carinii, Clostridium difficile,* Campylobacter species, Yersinia species, Shigella species and Listeria species. Viruses which are detectable include, but are not limited to, herpes, Epstein Barr virus, cytomegalovirus, human papilloma virus, influenza viruses, respiratory syncytial viruses, hepatitis and retroviruses (such as HTLV-I, HIV-I and HIV-II). Protozoan parasites, fungi (including yeasts) and molds are also detectable. Other detectable species would be readily apparent to one skilled in the art. The invention is particularly useful for the detection of the presence of DNA associated with various bacteria or viruses.

As used herein in referring to primers or probes, the term "oligonucleotide" refers to a molecule comprised of two or more deoxyribonucleotides or ribonucleotides, and preferably more than three. Its exact size is not critical but depends upon many factors including the ultimate use or function of the oligonucleotide. The oligonucleotide may be derived by any method known in the art.

A "PCR reagent" refers to any of the reagents considered essential to PCR, namely one or more primers for the target nucleic acid, a DNA polymerase, a DNA polymerase cofactor, and one or more deoxyribonucleotide-5'-triphosphates.

The term "primer" refers to an oligonucleotide, whether naturally occurring or synthetically produced, which is capable of acting as a point of initiation of synthesis when placed under conditions in which synthesis of a primer extension product complementary to a nucleic acid strand (that is, template) is induced. Such conditions include the presence of nucleotides (such as the four standard deoxyribonucleoside-5'-triphosphates), a DNA polymerase, and suitable temperature, pH and cofactor.

The primer is long enough to prime the synthesis of extension products in the presence of the DNA polymerase. The exact size of each primer will vary depending upon the use contemplated, the complexity of the targeted sequence, reaction temperature and the source of the primer. Generally, the primers used in this invention will have from 12 to 60 nucleotides, and preferably, they have from 18 to 45 nucleotides.

Primers useful herein can be obtained from a number of sources or prepared using known techniques and equipment, including for example, an ABI DNA Synthesizer (available from Applied Biosystems) or a Biosearch 8600 Series or 8800 Series Synthesizer (available from Milligen-Biosearch, Inc.) and known methods for their use (for example as described in US-A-4,965,188). Naturally occurring primers isolated from biological sources are also useful (such as restriction endonuclease digests). As used herein, the term "primer" also refers to a mixture of primers.

Each primer is labeled with the same label for detection. Procedures for attaching labels and preparing primers are well known in the art, for example, as described by Agrawal et al, Nucleic Acid Res., 14, pp. 6227-45 (1986), US-A-4,914,210 relating to biotin labels, US-A-4,962,029 relating to enzyme labels, and the references noted therein. Useful labels also include radioisotopes, electron-dense reagents, chromogens, fluorogens, phosphorescent moieties, ferritin and other magnetic particles (see US-A-4,795,698 and US-A-4,920,061), chemiluminescent moieties (such as luminol), and other specific binding species (avidin, streptavidin, sugars or lectins). Preferred labels are enzymes, radioisotopes and specific binding species (such as biotin). Useful enzymes include, glucose oxidase, peroxidases, uricase, alkaline phosphatase and others known in the art and can be attached to oligonucleotides using known procedures. Reagents to provide a colorimetric or chemiluminescent signal with such enzymes are well known.

Where the label is an enzyme such as a peroxidase, at some point in the assay, hydrogen peroxide and suitable dye-forming compositions are added to provide a detectable dye. For example, useful dye-providing reagents include tetramethylbenzidine and derivatives thereof, and leuco dyes, such as water-insoluble triarylimidazole leuco dyes (as described in US-A-4,089,747), or other compounds which react to provide a dye in the presence of peroxidase and hydrogen peroxide. Particularly useful dye-providing compositions are described in EP-A-0 308 236.

In a preferred embodiment, both primers are biotinylated and the amplified nucleic acid is detected using detectably labeled avidin or a derivative thereof (such as streptavidin). For example, avidin can be conjugated with an enzyme, or have a radioisotope using known technology. Biotin on the amplified product complexes with the avidin, and appropriate detection techniques to detect a radioactive, colorimetric or chemiluminescent signal are used.

A DNA polymerase is an enzyme which will add deoxynucleoside monophosphate molecules to the 3' hydroxy end of the primer in a complex of primer and template, but this addition is in a template dependent manner (that is, dependent upon the specific nucleotides in the template). Many useful DNA polymerases are known in the art. Preferably, the polymerase is "thermostable", meaning that it is stable to heat and preferentially active at higher temperatures, especially the high temperatures used for denaturation of DNA strands. More particularly, the thermostable DNA polymerases are not substantially inactivated by the high temperatures used in PCR as described herein. Such temperatures will vary depending upon a number of reaction conditions, including pH, the nucleotide composition of the target nucleic acid and primers, the length of primer, salt concentration and other conditions known in the art.

As used herein, a capture "probe" is an oligonucleotide which is substantially complementary to a nucleic acid sequence of one or more strands of the target nucleic acid, and which is used to insolubilize the amplified nucleic acid. The probe oligonucleotide is generally attached to a suitable water-insoluble substrate such as polymeric or glass beads, microtiter plate well, thin polymeric or cellulosic film or other materials readily apparent to one skilled in the art. The oligonucleotide is generally from 12 to 40 nucleotides in length, although the length is not critical.

A number of thermostable DNA polymerases have been reported in the art, including those mentioned in detail in US-A-4,965,188 and US-A-4,889,818. particularly useful polymerases are those obtained from various Thermus bacterial species, such as *Thermus aquaticus, Thermus thermophilus, Thermus filiformis* or *Thermus flavus.* Other useful thermostable polymerases are obtained from a variety of other microbial sources including *Thermococcus literalis, Pyrococcus furiosus,* Thermotoga sp. and those described in WO-A-89/06691. Some useful polymerases are commercially available. A number of techniques are known for isolating naturally-occurring polymerases from organisms, and for producing genetically engineered enzymes using recombinant techniques, as noted in the art cited in this paragraph. A preferred method for preparing a DNA polymerase equivalent to that obtained from *Thermus aquaticus* is described in EP-A-0 482 714.

A DNA polymerase cofactor refers to a nonprotein compound on which the enzyme depends for activity. Thus, the enzyme is catalytically inactive without the presence of the cofactor. The exact mechanism of the interaction of the cofactor with the polymerase is unknown at present. A number of such materials are known cofactors including manganese and magnesium compounds. Such compounds contain the manganese or magnesium in such a form that divalent cations are released into an aqueous solution. Useful cofactors include, but are not limited to, manganese and magnesium salts, such as chlorides, sulfates, acetates and fatty acid salts (for example, butyric, caproic, caprylic, capric and lauric acid salts). The smaller salts, that is chlorides, sulfates and acetates, are preferred.

Magnesium salts, such as magnesium chlorides and sulfates are most preferred in the practice of the invention.

Also needed for PCR is a deoxyribonucleotide-5'-triphosphate, such as dATP, dCTP, dGTP, dUTP or dTTP. Usually, dATP, dCTP, dGTP and dTTP are all used in PCR. Analogues such as dITP and 7-deaza-dGTP are also useful.

Each PCR reagent can be supplied individually packaged, or in a mixture with one or more other PCR reagents, including primers, DNA polymerase cofactors and deoxyribonucleotide-5'-triphosphates, all in a suitable buffer. Representative buffers include, but are not limited to, tris(hydroxymethyl)aminomethane (which is preferred), N,N-bis(2-hydroxyethyl)-2-aminoethanesulfonic acid, 4-(2-hydroxyethyl)-1-piperazinethanesulfonic acid, N-(2-hydroxyethyl)piperazine-N'-(2-hydroxypropanesulfonic acid), 3-(N-morpholino)propanesulfonic acid and N-[tris(hydroxymethyl)methyl]-2-aminoethanesulfonic acid.

A target nucleic acid (that is, one to be amplified or detected) can be obtained from any of a variety of sources as noted above. Generally, it is extracted in some manner to make it available for contact with the primers and other PCR reagents. This usually means removing unwanted proteins and cellular matter from the specimen in a suitable manner. Various procedures are known in the art, including those described by Laure et al in The Lancet, pp. 538-540 (Sept. 3, 1988), Maniatis et al, Molecular Cloning: A Laboratory Manual, pp. 280-281 (1982), Gross-Belland et al in Eur.J.Biochem., 36, 32 (1973) and US-A-4,965,188. Extraction of DNA from whole blood or components thereof are described, for example, in EP-A-0 393 744, Bell et al, Proc. Natl. Acad. Sci. USA, 78(9), pp. 5759-5763 (1981) and Saiki et al, Bio/Technology, 3, pp. 1008-1012 (1985).

Since the nucleic acid to be amplified or detected is usually in double stranded form, the two strands must be separated (that is, denatured before priming can take place). This can occur during the extraction process, or be a separate step afterwards. Denaturation is accomplished using a heat treatment alone or in combination with any suitable other physical, chemical or enzymatic means as described in the art. Initial denaturation is generally carried out by heating the specimen suspected of containing the targeted nucleic acid at a first temperature of from 85 to 100°C for a suitable time, for example from 1 second to 3 minutes.

The denatured strands are then cooled to a temperature which is generally in the range of from 55 to 70°C. The time needed for cooling the denatured strands will vary depending upon the type of apparatus used for the PCR process.

Once the denatured strands are cooled, the reaction mixture containing PCR reagents is incubated at a suitable temperature to effect formation of primer extension products. Generally, this temperature is at least 50°C, and preferably in the range of from 65 to 75°C. The time for incubation can vary widely depending upon the incubation temperature, but in preferred embodiments, it is from 1 to 120 seconds.

The primer extension products thus formed can be detected in a suitable manner while as hybridized products, or denatured either for detection of one or both strands, or further cycling in PCR.

If the hybridized primer extension products are denatured, PCR can be carried out further in as many cycles of forming primer extension products and denaturation as desired. Generally, at least 20 cycles will be carried out, with from 20 to 50 cycles being preferred. The two labeled primers are used in each cycle.

After denaturation the last time in the assay, the final primer extension products can be detected, as described below.

The amplification method of this invention is preferably conducted in a continuous, automated manner so that the reaction mixture is temperature cycled in a controlled manner for desired preset times. A number of instruments have been developed for this purpose, as one of ordinary skill in the art would know.

One such instrument for this purpose is described in some detail in US-A-4,965,188 and EP-A-0 236 069, and involves moving liquids from one temperature environment to another under controlled conditions.

Another instrument utilizes temperature cycling without a liquid handling system, and is described in some detail in US-A-4,965,188 and EP-A-0 236 069. Generally, this instrument includes a heat conducting container for holding a number of reaction tubes containing reaction mixture, a means for heating, cooling and temperature maintenance, and a computing means to generate signals to control the amplification sequence, changes in temperature and timing.

A gas chromatograph has also been used for amplification, as described for example by Hoffman et al, Biotechniques, 6(10), pp. 932-936 (1988), and amplification in a "teacup" has been described as a simple and inexpensive technique [Innis et al (Eds.), PCR Protocols: A Guide to Methods and Applications, Chapter 51, pp. 429-434 by Robert Watson, Academic Press, Inc., 1990].

A preferred instrument for processing amplification reactions in a disposable chemical test pack is described in some detail in EP-A-O 402,994. In general, this instrument comprises a surface for supporting a chemical test pack, pressure applicators supported above the surface for acting on the reaction pack to transfer fluids between adjacent chambers in the test pack, and means for operating the pressure applicators through a range of movement extending across the test pack.

EP-A-0 402,994 provides details of useful chemical test packs which can be processed using the instrument described in that same publication. Also described therein are means for heating and cooling the test pack at repeated intervals (that is, through cycles) appropriate for the method of the present invention. As noted above, while these instruments and test packs are preferred in practicing the present invention, they are not considered essential to obtaining the beneficial results noted herein.

It is also useful for the method of this invention to be carried out in a suitable container. The most crude container would be a test tube, cuvette, flask or beaker, but more sophisticated containers have been fashioned in order to facilitate automated procedures for performing the method (see for example, WO-A-91/12342). For example, cuvette and chemical test packs (also known as pouches), constructed to provide certain temperature characteristics during the practice of the method, are described in US-A-4,902,624 and EP-A-0 381,501. Such test packs have a multiplicity of reaction chambers having various reagents, buffers and other materials which are useful at various stages in the amplification or detection method. The packs can be appropriately and rapidly heated and cooled in cycles to promote the various steps of the amplification method of this invention. Other useful containers could be suitably fashioned for automated or single use of the method of this invention.

In order for the amplified product to be detected, it is often useful (but not necessary) for it to be separated from the other materials in the reaction medium. This is done by any of a number of ways, but preferably by using a water-insoluble capture probe so that the primer extension products which are replicated in the method are water-insolubilized and removed from the reagent mixture. Probes can be attached to insoluble materials in a suitable manner.

The amplified product can be separated from undesired materials by using an oligonucleotide complementary thereto, which oligonucleotide is attached to an insoluble substrate (such as polymeric or magnetic particles) using known attachment techniques to form the capture probe (noted above). One such technique is described in EP-A-0 439,222. Other techniques are described for example in US-A-4,713,326, WO-A-88/01302 and EP-B-0 070 687 whereby intermediate oligonucleotides are used in a hybridized product of multiple components to which the capture oligonucleotide and amplified nucleic acid are joined. Separation can be achieved by centrifugation or subjecting the mixture to a magnetic field.

Other useful separation means are microporous filtration membranes such as the polyamide membranes marketed by Pall Corp. (for example as LOPRODYNE™ or BIODYNE™ membranes). They can be used uncoated or precoated with surfactants or other materials which facilitate the analytical procedures.

The membranes can be used as a separate substrate with suitable containers for carrying out other steps of the assay. They can be mounted as part of a disposable test device. Various disposable test devices are known in the art including those described in US-A-3,825,410, US-A-3,888,629, US-A-3,970,429 and US-A-4,446,232. Particularly useful devices are described in US-A-4,921,677.

Any useful solid support can be used for separation of water-insoluble product for detection, including a microtiter plate, test tube, beaker, beads, film, membrane filters, filter papers, gels, magnetic particles or glass wool. It can be made of a number of materials including glass, ceramics, metals, naturally occurring or synthetic polymers, cellulosic materials, filter materials and others readily apparent to one of ordinary skill in the art. Particularly useful solid support materials are polymeric beads generally having an average particle size of from 0.1 to 10 µmeters.

The detection can also be carried out by immobilizing a capture probe on a flat substrate, such as the microporous filtration membranes described above, or on thin polymeric films, film laminates, uncoated papers or polymer coated papers, a number of which are known in the art. Other details of such materials are provided in European Application 0 408 738.

The following examples are included to illustrate the practice of this invention, and are not meant to be limiting in any way. All percentages are by weight unless otherwise noted.

### Materials and Methods for Examples:

Recombinant DNA polymerase from *Thermus aquaticus* was prepared as described in EP-A- 482,714 and had an activity of 250,000 units/ mg of protein. The activity can vary depending upon the source and procedure for obtaining the enyzme.

The primers and probes used in Example 1 were prepared using known starting materials and procedures using a BIOSEARCH™ Model 8700 DNA synthesizer and had the following sequences: 
SEQ ID NO:1:
5'-X-CAGCACCATC CTCCTCTTCC TCTGG-3'
SEQ ID NO:2:
5'-X-GAGGCTATTG TAGCCTACAC TTTGG-3'
The two previous primers are complementary to complementary strands of a nucleic acid sequence in the "major immediate early" MIE gene of cytomegaloviral DNA. The two following primers are complementary to complementary strands of a nucleic acid sequence in the "late" (LA) gene of cytomegaloviral DNA. 
SEQ ID NO:3:
5'-X-GTCGCCTGCA CTGCCAGGTG CTTCG-3'
SEQ ID NO:4:
5'-X-CACCACGCAG CGGCCCTTGA TGTTT-3'
In these sequences, X represents a biotin moiety attached to the sequence through two tetraethylene glycol spacer units and a 3-amino-1,2-propanediol using the teaching of US-A-4,914,210.

The oligonucleotides used in the capture probes were as follows: 
SEQ ID NO:5:
5'-Y-GGTGTCACCC CCAGAGTCCC CTGTACCCGC-3'
SEQ ID NO:6:
5'-Y-GTGGAAGGCG GCTCGCTGGA AGCCGGTCGT-3'
wherein Y is a polyethylene glycol (molecular weight of 600) spacer and a 3-amino-1,2-propanediol moiety prepared using the procedures of the Levenson et al patent US-A-4,914,210 noted above.

The first oligonucleotide was directed to the MIE gene sequence while the second oligonucleotide was directed to the LA gene sequence.

Additional capture probes for Example 2 were also prepared, having oligonucleotides with the following sequences: 
SEQ ID NO:7:
5'-GACACAGTGT CCTCCCGCTC CTCCTGAGCA-Y-3'
SEQ ID NO:8:
5'-GAACCGAGGG CCGGCTCACC TCTATGTTGG-Y-3'
The first capture probe oligonucleotide is complementary to a MIE gene sequence, while the second is complementary to the LA gene sequence of cytomegaloviral DNA.

### Example 1 Amplification and Detection of DNA Associated with Cytomegalovirus

This example demonstrates the practice of this invention to amplify and detect DNA associated with cytomegalovirus (CMV) using two sets of labeled primers. It also shows a comparison of the practice of this invention with a Control experiment where only one primer of one primer set was labeled.

Purified CMV DNA from the Towne strain (1:500 dilution) was amplified in a microcentrifuge tube (250 µl volume) using a commercially available Perkin Elmer THERMOCYCLER™. The four primers (1 µmolar each of SEQ ID NO:1 through SEQ ID NO:4) were mixed with tris(hydroxymethyl)aminomethane hydrochloride buffer (10 mmolar), potassium chloride (50 mmolar), magnesium chloride (10 mmolar), gelatin (100 µg/ml), *Thermus aquaticus* DNA polymerase identified above (10 units) and dNTP's (1.5 mmolar of each) to form a PCR reagent composition.

PCR was carried out with 33 cycles of the following sequence of steps:
denaturing at 98°C for 30 seconds,
priming target nucleic acid strands at 55°C for 90 seconds, and
forming primer extension products at 72°C for 120 seconds.

Primer extension in the last cycle was carried out at 70°C for 15 minutes. The noted times in the PCR process do not include times to move from one temperature to another of 15 to 60 seconds. The times for cooling were slightly longer than the times for heating to higher temperatures.

The resulting amplified target nucleic acid was detected by capturing the biotinylated products by hybridization with capture probes which had been immobilized in a distinct area of a LOPRODYNE™ microporous polyamide membrane (5 µm pore size, Pall Corp.) in a test well of a commercially available SURECELL™ disposable test device (Eastman Kodak Company). The probes were composed of the oligonucleotides identified above as SEQ ID NO:5 and SEQ ID NO:6 covalently attached to particles (1.4 µm average diameter) of poly[styrene-co-3-(p-vinylbenzylthio)propionic acid] (97.6:2.4 molar ratio) using 1-ethyl-3-(3-dimethylaminopropyl)carbodiimide. Each probe had been deposited in distinct regions on the membranes as 0.25% dispersions in 2 µl and allowed to dry for 30 minutes.

The amplified products (1:20 dilution, 5 µl) were applied to the test well containing the capture probes in a 1:10 dilution of tris(hydroxymethyl)aminomethane hydrochloride (10 mmolar, pH 8) buffer (95 µl) containing the chlorides and gelatin noted above after a final denaturation at 95°C for 5 minutes. The products were allowed to hybridize to the capture probes for 5 minutes at 42°C. The membranes were then washed at 55°C with wash solution of a 1:10 dilution of SSPE buffer (Sigma Chemical Co., 250 µl) which also contained decyl sodium sulfate (1%).

A composition (50 µl) containing streptavidin-horseradish peroxidase conjugate (126 µl/l), casein (0.5%) and merthiolate preservative (0.5%) was added to the test well and allowed to react with the biotinylated product on the membrane at room temperature for two minutes. Unbound materials were washed away with the wash solution (250 µl) at 55°C.

The capture product on the particles was then contacted with a dispersion (100 µl) of 4,5-bis(4-methoxyphenyl)-2-(3,5-dimethoxy-4-hydroxyphenyl)imidazole leuco dye prepared by dispersing the leuco dye (to make a 0.1% solution) in a solution of poly(vinyl pyrrolidone) (20%) in sodium phosphate buffer (5 mmolar), and adding the resulting dispersion to a solution of hydrogen peroxide (10 mmolar), 4'-hydroxyacetanilide (5 mmolar) and diethylenetriaminepentaacetic acid (10 µmolar) in sodium phosphate buffer to have a final concentration of 1% polymer and 0.005% leuco dye.

Dye formation was allowed to proceed for two minutes, after which a solution (100 µl) of sodium azide (0.1%) was added. The visible dye density on the membrane was then compared to the calibrated color density scores from 0 (no dye) to 10 (highest density).

The Control experiments were carried out in similar fashion except that only one biotinylated primer for the MIE gene sequence was used (SEQ ID NO:1 was used without the biotin moiety).

The results of two assays carried out simultaneously are provided in the following table. Controls 1 and 2 were separate runs of the same experiments.

**TABLE 1**

| Assay | Color Scores | |
|---|---|---|
| | MIE Probe | LA Probe |
| Control 1 | 3/3 | 6/6 |
| Control 1 (duplicate) | 4/4 | 7/6.5 |
| Control 2 | 3/3.5 | 7/7 |
| Control 2 (duplicate) | 3.5/4 | 7/7 |
| Example 1 | 6/6 | 7/7 |
| Example 1 (duplicate) | 6/6 | 7/7 |

### Example 2 Amplification and Detection Using Capture Probes for Each Amplified Strand

This example is similar to Example 1 except that a capture probe was used for each strand of amplified target nucleic acid sequence.

The microporous membrane in test wells of SURECELL™ disposable test devices were similarly prepared except that a mixture of dispersions of capture probes having oligonucleotides SEQ ID NO:5 and SEQ ID NO:7 (0.125% of each) was applied and dried in a distinct region thereof. A mixture of the capture probes having oligonucleotides SEQ ID NO:6 and SEQ ID NO:8 (0.125% of each) was deposited and dried in a different region of the membrane.

The results of two assays carried out simultaneously, and duplicates carried out using the same target nucleic acid sample (1:500 dilution) on a different day, are shown in the following Table II. These results indicate that the use of a capture probe for each amplified target nucleic acid strand provided excellent sensitivity.

**TABLE II**

| MIE Probes | Color Scores | |
|---|---|---|
| | Day 1 | Day 2 |
| SEQ ID NO.: 5 | 7/7 | 4.5/4.5 |
| SEQ ID NO.: 7 | 7/7.5 | 6/6 |
| Both | 7/6 | 4/3 |
| SEQ ID NO.: 6 | 6.5/6.5 | 5/5 |
| SEQ ID NO.: 8 | 7/7.5 | 7/7 |
| Both | 7.5/7 | 5/5 |

## Claims

1. A method for the production of a target nucleic acid comprising:
amplifying the denatured strands of a target nucleic acid in the presence of two primers which are complementary to the complementary strands of the target nucleic acid, one or more deoxyribonucleotide-5'-triphosphates, a DNA polymerase and a DNA polymerase cofactor,
both of the primers being labeled with the same detection moiety.

2. A method for the amplification of a target nucleic acid comprising:
A) contacting the denatured strands of a target nucleic acid in the presence of polymerase chain reaction reagents comprising: two primers which are complementary to the complementary strands of the target nucleic acid, one or more deoxyribonucleotide-5'-triphosphates, a DNA polymerase and a DNA polymerase cofactor,
both of the primers being labeled with the same detection moiety, and
B) forming primer extension products of the target nucleic acid strands with the labeled primers.

3. A method for the detection of a target nucleic acid comprising:
A) contacting the denatured strands of a target nucleic acid with the polymerase chain reaction reagents comprising: two primers which are complementary to the complementary strands of the target nucleic acid, one or more deoxyribonucleotide-5'-triphosphates, a DNA polymerase and a DNA polymerase cofactor,
both of the primers being labeled with the same detection moiety,
B) forming primer extension products of the target nucleic acid strands with the labeled primers,
C) denaturing the strands of the resulting primer extension products,
D) repeating steps B) and C) as a PCR cycle at least one more time, and
E) detecting at least one of the denatured strands after the last PCR cycle.

4. The method as claimed in claim 3 for the detection of a viral, bacterial or fungal RNA or DNA or the RNA or DNA associated with any disease state.

5. The method as claimed in either of claims 3 or 4 wherein step D) is carried out at least 20 times.

6. The method as claimed in any of claims 1 to 5 wherein each of the primers is labeled with an enzyme, radioisotope or specific binding species.

7. The method of claim 6 wherein each of the primers is labeled with biotin.

8. The method of claim 7 wherein the amplified biotinylated target nucleic acid is detected using a conjugate of avidin or a derivative thereof, and an enzyme.

9. A kit for polymerase chain reaction comprising, in separate packaging:
a) a set of two primers complementary to the complementary strands of a target nucleic acid, both of the primers being labeled with the same detection moiety, and
b) at least one additional PCR reagent.

10. The kit of claim 9 wherein each of the primers is labeled with biotin, and the kit comprises additionally a DNA polymerase which is isolated from a Thermus species or is a genetically engineered equivalent thereof, a DNA polymerase cofactor which is a magnesium or manganese salt, and dATP, dCTP, dGTP and dTTP.
